# FASCICULE DE BREVET EUROPEEN

(11) **EP 0 662 807 B1**
(45) Date de publication et mention de la délivrance du brevet: **26.11.1997**
(21) Numéro de dépôt: 93921971.3
(22) Date de dépôt: 30.09.1993
(51) Int. Cl.: A61F 2/10

(54) **DISPOSITIF POUR TRANSPLANTATION DE GREFFONS CAPILLAIRES DE PETIT DIAMETRE**
VORRICHTUNG ZUR IMPLANTATION VON HAARTRANSPLANTAT MIT KLEINEM DURCHMESSER
DEVICE FOR TRANSPLANTING SMALL DIAMETER HAIR GRAFTS

(30) Priorité: 01.10.1992 FR 9212105
(43) Date de publication de la demande: 19.07.1995
(73) Titulaire: MEDICAMAT S.A., F-92240 Malakoff (FR)
(72) Inventeur: Boudjema, Jamil Pascal, F-75016 Paris (FR)
(74) Mandataire: Plaçais, Jean-Yves
(86) Numéro de dépôt international: FR9300955
(87) Numéro de publication internationale: WO9407433

(56) Documents cités:
- EP-A- 0 237 818
- WO-A-91/13596
- US-A- 4 838 853
- US-A- 4 867 155

## Description

La présente invention concerne un dispositif pour transplantation de greffons capillaires de petit diamètre dans le cuir chevelu, comprenant un instrument de coupe chirurgical comportant un outil cylindrique rotatif muni d'un embout de forage creux cylindrique de découpe des greffons à une extrémité.

Elle trouve une application particulièrement importante bien que non exclusive dans le domaine du traitement chirurgical de la calvitie par greffe des cheveux naturels, et plus particulièrement dans le domaine de la micro-greffe.

Le traitement de la calvitie par transplantation capillaire, dite technique des greffons, consiste à transplanter chez un même individu, une partie des racines de ses cheveux situées dans la couronne (toujours chevelue) vers les zones chauves.

Les greffons sont des fragments cutanés cylindriques obtenus par découpe circulaire de la peau à l'aide d'un instrument de coupe chirurgical tel que défini ci-dessus.

Les fragments cutanés ainsi découpés sur leur périphérie sont, dans un second temps, prélevés un par un à l'aide de pinces et de ciseaux, puis, dans un troisième temps, sont réimplantés un par un dans des sites receveurs respectifs préalablement préparés.

Les techniques chirurgicales connues découpent des greffons de diamètre de plus en plus réduit, de micro-greffons par exemple, d'environ 1,5 mm de diamètre et 6 mm de long, afin de garantir un résultat esthétique de grande qualité.

Malheureusement, ces micro-greffons restent souvent bloqués au fond de l'embout de forage du fait de leur très faible diamètre, ce qui nécessite de les déloger manuellement à l'aide d'une micro-pince.

Indépendamment de cet inconvénient, les micro-greffons découpés doivent être saisis un à un à l'aide de micro-pinces, détachés du cuir chevelu avec des ciseaux, puis placés dans une solution de conservation afin d'éviter leur dessication pendant la préparation des sites receveurs dans lesquels ils sont finalement placés manuellement un à un à l'aide de micro-pinces.

Ces manipulations fastidieuses et très délicates risquent de traumatiser les micro-greffons et allongent considérablement les temps opératoires.

La présente invention vise à éliminer les inconvénients énumérés ci-dessus en proposant un dispositif répondant mieux que ceux antérieurement connus aux exigences de la pratique, notamment en permettant de prélever rapidement des micro-greffons d'excellente qualité, puis de les réimplanter ensuite immédiatement ou presque immédiatement après leur prélèvement, pour un coût faible, de façon simple et aisée à mettre en oeuvre.

Dans ce but, l'invention propose essentiellement un dispositif comme défini dans la revendication 1.

Dans des modes de réalisation avantageux, on a de plus recours à l'une et/ou à l'autre des dispositions suivantes :
- le tuyau collecteur flexible est de même diamètre interne ou sensiblement de même diamètre interne que l'alésage de l'aiguille, et les moyens de prélèvement comprennent des moyens de connexion étanche du tuyau collecteur à l'extrémité arrière de l'aiguille rotative, et des moyens de mise au vide de l'alésage de l'aiguille et du tuyau collecteur.
- les moyens de connexion comprennent un conduit solidaire du corps, raccordé axialement d'un côté avec le tuyau flexible, l'extrémité arrière de l'aiguille coopérant rotativement et de façon étanche avec l'autre extrémité dudit conduit, ledit conduit comprenant un alésage interne de même diamètre interne que le diamètre interne du tuyau collecteur;
- l'aiguille est montée de façon amovible par rapport au corps de l'instrument de coupe;
- le dispositif comporte de plus un appareil portatif d'implantation du greffon prélevé, l'appareil comprenant un corps, le réservoir de stockage intermédiaire de greffon, une seconde aiguille creuse, de réimplantation, percée de part en part d'un alésage de même diamètre que l'alésage de la première aiguille creuse, et des moyens d'introduction du greffon dans le cuir chevelu au travers de ladite seconde aiguille creuse, à partir du réservoir intermédiaire;
- le réservoir de stockage intermédiaire du greffon comprend un premier canal connecté axialement à la seconde aiguille creuse;
- le premier canal est cylindrique, de même diamètre ou sensiblement de même diamètre que le tuyau flexible avec lequel il est connecté obliquement;
- le premier canal est rectiligne et les moyens d'introduction du greffon dans le cuir chevelu comprennent un premier piston propre à coopérer avec l'alésage interne de la seconde aiguille, agencé pour pousser le greffon au travers de la seconde aiguille;
- la connexion oblique présente une pente dirigée de l'avant vers l'arrière de la seconde aiguille, et comprise entre de l'ordre de 10° et de l'ordre de 60°;
- les moyens d'introduction sont pneumatiques;
- les moyens d'introduction sont manuels;
- l'appareil comporte de plus des moyens de butée contre le cuir chevelu agencés pour provoquer le retrait de la seconde aiguille après introduction et dépose du greffon dans le cuir chevelu;
- les moyens de butée comportent un second canal, parallèle au premier canal, et un second piston propre à venir prendre appui sur le cuir chevelu, le second piston étant solidaire du premier piston.

De nombreux avantages et caractéristiques de la présente invention ressortiront au cours de la description détaillée qui va suivre, faite en regard des dessins annexés donnant à titre explicatif mais nullement limitatif une forme de réalisation conforme à l'invention.

Sur ces dessins :
- la figure 1 est une coupe longitudinale de l'instrument de coupe appartenant au dispositif selon un mode de réalisation de l'invention;
- la figure 2 est une coupe longitudinale verticale d'un appareil de réimplantation, en forme de pistolet, selon un mode de réalisation de l'invention;
- la figure 3 est une coupe longitudinale horizontale suivant la ligne III-III de la figure 2;
- la figure 4 est une vue de devant de l'appareil de réimplantation de la figure 2;
- la figure 5 est une coupe transversale selon la ligne V-V de la figure 2;
- la figure 6 est une coupe transversale selon la ligne VI-VI de la figure 2;
- la figure 7 est une vue représentant les liaisons entre l'instrument de coupe chirurgical et l'appareil de réimplantation avec le dispositif de commande et de mise sous vide.

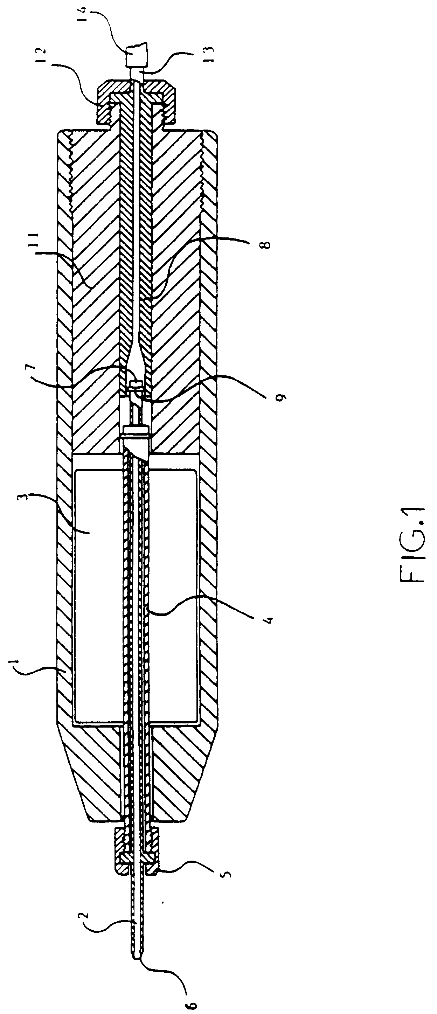

Sur la figure 1, on voit que l'instrument de coupe chirurgical selon l'invention est constitué d'un corps allongé 1 de forme cylindrique (dont les dimensions peuvent être d'environ 12 cm de long et 3 cm de diamètre) faisant office de pièce-à-main, dans lequel est disposée dans son axe une aiguille métallique creuse 2, cylindrique, droite, rigide à paroi très mince, de très faible diamètre intérieur (environ 1,5 mm), propre à être animée d'un mouvement rotatif axial par l'intermédiaire d'un moteur 3 à arbre d'entraînement creux 4 dont le diamètre intérieur est très légèrement supérieur au diamètre extérieur de l'aiguille 2.

L'aiguille 2 est maintenue solidairement dans l'arbre creux 4 par un écrou de serrage 5 disposé à l'avant et à l'extérieur du corps 1.

L'extrémité antérieure 6 de l'aiguille 2 dépassant de quelques centimètres, la tête de la pièce-à-main présente une section droite dont le bord circulaire biseauté et tranchant fait office d'extrémité de forage au niveau du cuir chevelu. L'aiguille 2 se prolonge à son extrémité postérieure 7, au sein de la pièce-à-main, par une aiguille métallique fixe 8, creuse, droite, cylindrique (aiguille collectrice) disposée dans son axe d'un diamètre légèrement supérieur au niveau de la jonction avec l'extrémité postérieure de l'aiguille 2 qui s'y encastre sur quelques millimètres, hermétiquement, par l'intermédiaire d'une bague à lèvre ou joint 9, étanche à l'air.

L'aiguille fixe 8 est disposée dans l'alésage axial central d'un corps cylindrique 11 vissé à l'arrière du corps 1. Elle est fixée au corps cylindrique 11 par un écrou de serrage 12 disposé à l'arrière et à l'extérieur du corps 1.

A l'extrémité postérieure 13 de l'aiguille 8 vient s'aboucher à l'extérieur de la pièce-à-main un tuyau en matière plastique souple 14 (tuyau collecteur) de même diamètre intérieur que celui de l'aiguille 8 et de longueur suffisante pour être raccordé au pistolet de réimplantation.

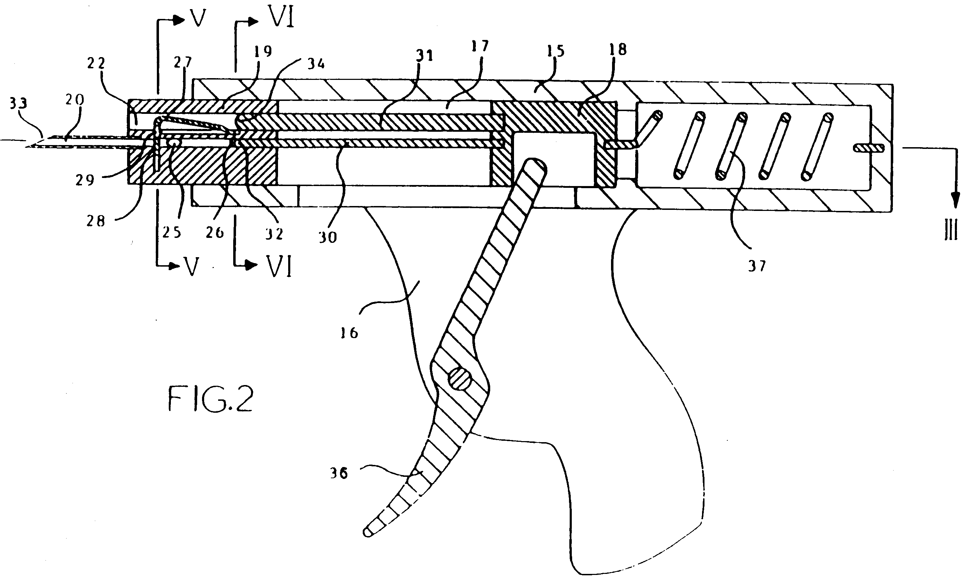

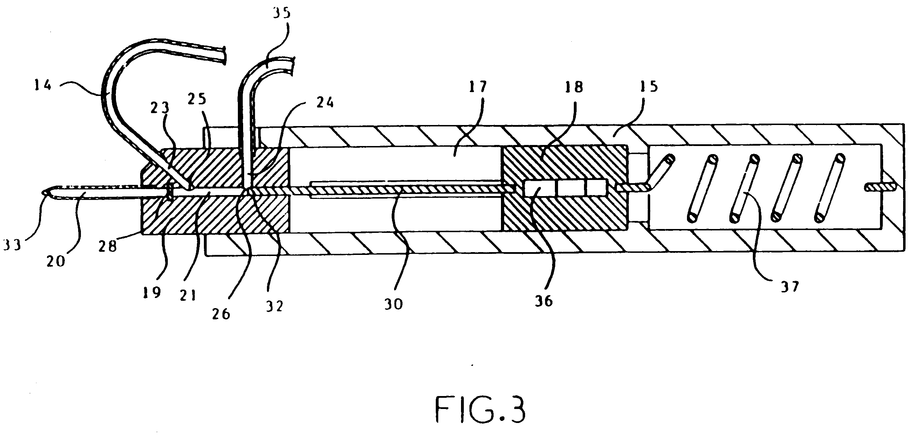

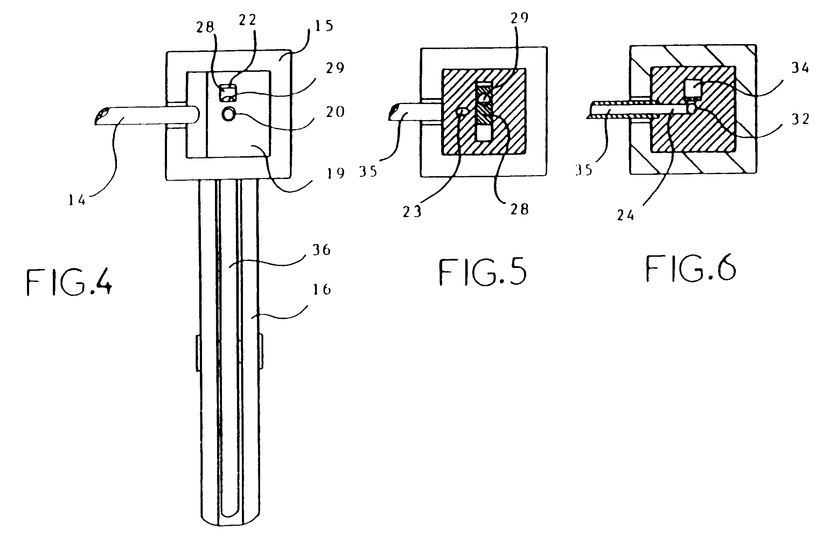

Sur les figures 2 à 6, on voit que le dispositif de réimplantation est constitué d'une pièce-à-main ayant la forme d'un pistolet présentant un corps 15 et une crosse 16.

Le corps du pistolet est constitué d'un cylindre creux 17 à l'intérieur duquel coulisse un piston 18 selon un mouvement de translation horizontal.

A l'extrémité antérieure du cylindre 17 vient s'encastrer un organe fixe 19 (tête de réimplantation) de forme sensiblement identique à celle du piston, prolongé en avant et à l'extérieur par une aiguille creuse 20 (aiguille de réimplantation d'environ 3 à 4 cm de longueur).

La tête de réimplantation 19 est creusée, le long de son axe et sur toute sa longueur, de deux canaux parallèles entre eux :
- un canal longitudinal central 21 (canal collecteur du greffon) ou premier canal; et
- un canal longitudinal paracentral 22 ou second canal.

Le canal central 21 est rectiligne, cylindrique, de même diamètre que le diamètre intérieur de l'aiguille de réimplantation 20 avec laquelle il se prolonge en avant.

Deux petits canaux cylindriques 23 et 24 rectilignes viennent s'aboucher latéralement dans le canal central 21.

Le canal antérieur 23 (canal d'arrivée du greffon) de même diamètre que le canal central 21 s'abouche dans ce dernier selon un angle suffisamment ouvert en arrière, de telle sorte qu'un greffon arrivant par le canal latéral antérieur 23, puisse aisément se positionner dans le canal central 21, sans être altéré.

Le canal latéral postérieur 24 (canal d'aspiration) s'abouche perpendiculairement dans le canal central 21.

Le canal latéral postérieur 24 est relié à l'extérieur à une source de vide connue en elle-même, par un tuyau en matière plastique souple 35.

La distance séparant les orifices d'abouchement des canaux latéraux 23 et 24 dans le canal central 21 doit être supérieure à la longueur d'un greffon (environ 6 mm).

Au sein du canal paracentral 22 est disposé un organe élastique à type de ressort à lame 27, prolongé à sa partie antérieure par une portion verticale 28 faisant office de clapet, venant en position de repos, obstruer hermétiquement le canal central 21, en avant de l'orifice d'abouchement 25.

Une ouverture 29 est disposée au sein de la portion verticale 28 du dispositif 27, de telle sorte que lorsque la portion verticale 28 est abaissée (par un moyen que nous décrirons plus loin), cette ouverture 29 puisse faire communiquer librement le canal central 21 avec l'aiguille 20.

Le piston 18 est constitué d'une pièce mobile dans le cylindre 17, prolongée en avant par deux tiges rigides rectilignes longitudinales parallèles entre elles et de même longueur : une tige centrale 30 et une tige paracentrale 31.

La tige centrale 30 (tige de réimplantation) de forme cylindrique est disposée dans l'axe du canal central 21 dans lequel elle est engagée à sa partie postérieure.

De la même manière, la tige paracentrale 31 (tige guide) est disposée dans l'axe du canal paracentral 22 dans lequel elle est engagée à sa partie postérieure.

Le diamètre et la longueur de la tige centrale 30 sont tels que lorsque le piston 18 est mobilisé en avant dans le cylindre 17, elle puisse coulisser librement et hermétiquement sur toute la longueur du canal central 21 et de l'aiguille 20, et que son extrémité antérieure 32 puisse dépasser légèrement l'extrémité 33 de l'aiguille 20, en bout de course.

La distance au repos séparant le piston 18 de la tête de réimplantation 19 est telle que la tige centrale 30 soit déjà engagée dans le canal central 21 et que son extrémité antérieure 32 vienne obstruer partiellement l'orifice d'abouchement 26 du canal latéral postérieur 24.

De la même manière, la tige paracentrale 31 est disposée de telle sorte qu'au repos elle soit déjà engagée dans le canal paracentral 22 et sans action sur le ressort à lame 27 et que lorsqu'elle est mobilisée en avant parallèlement et conjointement à la tige centrale 30, son extrémité antérieure 34 abaisse la lame 27 et sa portion verticale 28 et de ce fait, fait communiquer le canal central 21 avec l'aiguille 20 par l'ouverture 29.

En fin de course, l'extrémité antérieure 34 de la tige 31 est également conçue pour servir de butée contre la peau. La mobilisation avant du piston 18 dans le cylindre 17 est assurée manuellement par un levier ou gâchette 36. Le retour du piston 18 en arrière, en position de repos, est assuré par l'action d'un ressort 37.

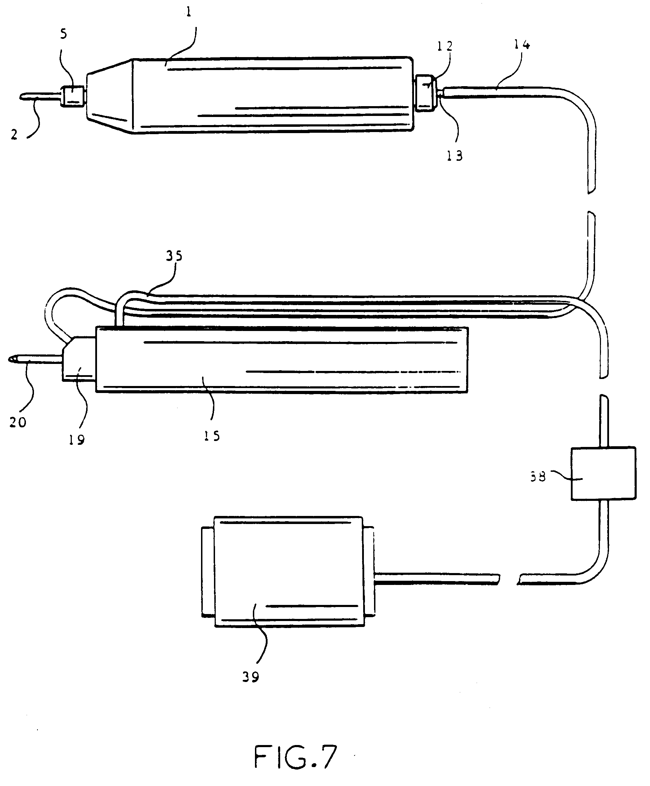

Sur la figure 7, on voit que le vide peut être transmis à volonté dans le pistolet de réimplantation et dans l'instrument de coupe par l'intermédiaire d'un dispositif de commande 38 relié à la pompe à vide 39.

Concernant l'instrument de coupe chirurgical selon l'invention, bien qu'un moteur électrique ait été représenté comme source de force motrice pour l'aiguille de forage, il est possible d'utiliser un moteur pneumatique ou un moteur a vide. Le corps de l'instrument de coupe peut être réalisé en matière plastique rigide de haute densité ou en métal (aluminium).

Concernant le dispositif de réimplantation, bien que la mobilisation du piston 18 dans le cylindre 17 ait été représentée par une force humaine exercée sur une gâchette 36, il est possible, par exemple, d'utiliser une source de force pneumatique.

Le corps 15 du pistolet de réimplantation peut avantageusement être réalisé en aluminium. La tête de réimplantation 19 peut être réalisée en métal (aluminium ou acier inoxydable) ou en matière plastique transparente rigide telle que le méthacrylate de méthyle.

Le dispositif à type de ressort 27 peut par exemple être réalisé en métal ou en matière plastique rigide. Les tiges 30 et 31 peuvent être réalisées en acier ou en matière plastique rigide de haute densité. Les tuyaux 14 et 35 peuvent par exemple être réalises en polyéthylène.

Bien que la tête de réimplantation 19 et le piston 18 aient été représentés de forme parallélépipédique sur les dessins, il est bien entendu également possible, par exemple, de les concevoir de forme cylindrique. De même, bien que le canal paracentral 22 dans lequel coulisse la tige 31 ait été représenté de section carrée sur les dessins, il est possible de le concevoir de forme cylindrique. L'extrémité 33 de l'aiguille de réimplantation 20 peut être biseautée ou de section droite.

Le dispositif selon l'invention fonctionne comme suit.

Il peut nécessiter la participation de deux opérateurs :
- un préleveur maintenant l'instrument de coupe chirurgical; et
- un implanteur maintenant le pistolet de réimplantation.

L'instrument de coupe chirurgical est mis en marche, alors que le vide est coupé.

L'aiguille de forage est introduite dans le cuir chevelu au niveau de la couronne jusqu'à une profondeur suffisante (environ 6 mm) pour englober une à quelques racines de cheveux. Le vide est alors ouvert par l'intermédiaire du dispositif de commande 38, ce qui a pour but d'aspirer, en une fraction de seconde, le greffon cutané dont les dimensions avoisinent 1,5 mm de diamètre et 6 mm de long qui va cheminer respectivement à l'intérieur de l'aiguille 2, dans l'aiguille collectrice 8, le tuyau collecteur 14, le canal latéral 23 pour finir sa course dans le canal central 21, en venant buter contre l'extrémité 32 de la tige de réimplantation 30, au sein du pistolet de réimplantation. Le vide est alors coupé.

L'implanteur introduit l'aiguille de réimplantation 20 au fond de l'orifice cutané receveur, de diamètre et de profondeur sensiblement identique au greffon, préalablement préparé au niveau de la zone chauve.

L'implanteur actionne alors le pistolet mobilisant en avant le piston 18 dans le cylindre 17. Le greffon est alors poussé en avant dans le canal central 21 par l'extrémité 32 de la tige 30.

Parallèlement, la tige paracentrale 31 abaisse le dispositif 27 au sein du canal 22, faisant communiquer le greffon en avant avec l'aiguille 20 par l'ouverture 29. Le greffon chemine alors jusqu'à l'extrémité 33 de l'aiguille 20. L'extrémité 34 de la tige paracentrale 31 située à la même hauteur que l'extrémité 32 de la tige de réimplantation 30 venant alors buter contre la surface cutanée avoisinant l'orifice dans lequel se trouve l'aiguille 20, va provoquer le recul de la tête de réimplantation 19 et par la même occasion le retrait simultané de l'aiguille 20, ce qui a pour conséquence de laisser en place le greffon dans son orifice receveur. L'opération est alors répétée de la même manière selon la même séquence pour chaque greffon.

## Revendications

1. Dispositif pour transplantation de greffons capillaires de petit diamètre comprenant un instrument portatif de coupe comportant un corps (1) porte-outil, un outil cylindrique rotatif (2) et un ensemble moteur (3) propre à entraîner ledit outil en rotation par rapport audit corps, ledit outil étant muni d'une extrémité creuse (6) de découpe d'un greffon, caractérisé en ce que l'outil cylindrique est une aiguille creuse percée de part en part d'un alésage axial de même diamètre que l'extrémité creuse de découpe qu'il prolonge, et en ce que le dispositif comporte des moyens (8,14,38,39) de prélèvement du greffon par aspiration dudit greffon au travers de l'alésage axial de l'aiguille, lesdits moyens de prélèvement étant munis d'un tuyau collecteur flexible (14) étant raccordé à un réservoir (21) de stockage intermédiaire (21) du greffon prélevé.

2. Dispositif selon la revendication 1, caractérisé en ce que :
ledit tuyau collecteur (14) flexible est de même diamètre interne ou sensiblement de même diamètre interne que l'alésage de l'aiguille,
et en ce que les moyens de prélèvement comprennent
des moyens (8,9) de connexion étanche dudit tuyau collecteur à l'extrémité arrière (17) de l'aiguille rotative,
et des moyens (38,39) de mise au vide de l'alésage de l'aiguille et du tuyau collecteur.

3. Dispositif selon la revendication 2, caractérisé en que les moyens (8,9) de connexion comprennent un conduit ( 8) solidaire du corps, raccordé axialement d'un côté (13) avec le tuyau flexible (14), l'extrémité arrière (7) de l'aiguille coopérant rotativement et de façon étanche avec l'autre extrémité dudit conduit, ledit conduit comprenant un alésage interne de même diamètre interne que le diamètre interne du tuyau collecteur.

4. Dispositif selon l'une quelconque des revendications précédentes, caractérisé en ce que l'aiguille (2) est montée de façon amovible par rapport au corps de l'instrument de coupe.

5. Dispositif selon l'une quelconque des revendications précédentes, caractérisé en ce qu'il comporte de plus un appareil portatif d'implantation du greffon prélevé, ledit appareil comprenant :
un corps (15),
le réservoir (21) de stockage intermédiaire de greffon,
une seconde aiguille creuse (20), de réimplantation, percée de part en part d'un alésage de même diamètre que l'alésage de la première aiguille creuse,
et des moyens d'introduction (18,30,36) du greffon dans le cuir chevelu au travers de ladite seconde aiguille creuse, à partir du réservoir intermédiaire.

6. Dispositif selon la revendication 5, caractérisé en ce que le réservoir (21) de stockage intermédiaire du greffon comprend un premier canal (21) connecté axialement à la seconde aiguille creuse.

7. Dispositif selon la revendication 6, caractérisé en ce que le premier canal (21) est cylindrique, de même diamètre ou sensiblement de même diamètre que le tuyau flexible (14) avec lequel il est connecté obliquement.

8. Dispositif selon la revendication 7, caractérisé en ce que le premier canal (21) est rectiligne et les moyens d'introduction du greffon dans le cuir chevelu comprennent un premier piston (30) propre à coopérer avec l'alésage interne de la seconde aiguille, agencé pour pousser ledit greffon au travers de la seconde aiguille.

9. Dispositif selon l'une quelconque des revendications 7 et 8, caractérisé en ce que la connexion (23) oblique présente une pente dirigée de l'avant vers l'arrière de la seconde aiguille, et comprise entre de l'ordre de 10° et de l'ordre de 60°.

10. Dispositif selon l'une quelconque des revendications 5 à 9, caractérisé en ce que les moyens d'introduction sont pneumatiques.

11. Dispositif selon l'une quelconque des revendications 5 à 9, caractérisé en ce que les moyens d'introduction sont manuels.

12. Dispositif selon l'une quelconque des revendications 5 à 11, caractérisé en ce que l'appareil comporte de plus des moyens (31,34) de butée contre le cuir chevelu agencés pour provoquer le retrait de la seconde aiguille (20) après introduction et dépose du greffon dans le cuir chevelu.

13. Dispositif selon la revendication 12 dépendante de la revendication 8, caractérisé en ce que les moyens de butée comportent un second canal (22) parallèle au premier canal (21) et un second piston (31) propre a venir prendre appui sur le cuir chevelu, le second piston étant solidaire du premier piston.

## Claims

1. Device for transplanting small diameter capillary grafts comprising a portable cutting device with a tool bearing body (1), a cylindrical rotating tool (2) and a motor assembly (3) able to drive the said tool in rotation in relation to the said body, the said tool being fitted with a hollow extremity (6) for cutting a graft, characterised in that the cylindrical tool is a hollow needle pierced through with an axial bore of the same diameter as the hollow cutting end which it extends, and in that the device comprises means (8, 14, 38, 39) for taking the graft by suction of the said graft through the axial bore of the needle, the said means of removal being fitted with a flexible collector hose (14) connected to a reservoir (21).

2. Device according to claim 1, characterised in that the said flexible collector hose (14) is of the same or approximately the same internal diameter as the bore of the needle,
and in that the means of taking comprise means (8, 9) for sealed connection of the said collector hose to the rear extremity (17) of the rotating needle,
and means (38, 39) for evacuating the bore of the needle and the collector hose.

3. Device according to claim 2, characterised in that the means (8, 9) of connection comprise a duct (8) of one piece with the body, connected axially on one side (13) to the flexible hose (14), the rear end (7) of the needle cooperating rotationally and in a sealed manner with the other end of the said duct, the said duct comprising an internal bore of the same internal diameter as the internal diameter of the collector hose.

4. Device according to any of the previous claims, characterised in that the needle (2) is mounted removably in relation to the body of the cutting instrument.

5. Device according to any of the previous claims, characterised in that it also comprises a portable device for implantation of the removed graft, the said device comprising:
a body (15),
the intermediate graft storage reservoir (21),
a second hollow needle (20) for re-implantation pierced through with a bore of the same diameter as the bore of the first hollow needle,
and means of insertion (18, 30, 36) of the graft in the scalp through the said second hollow needle from the intermediate reservoir.

6. Device according to claim 5, characterised in that the intermediate graft storage reservoir (21) comprises a first channel (21) connected axially to the second hollow needle.

7. Device according to claim 6, characterised in that the first channel (21) is cylindrical of the same diameter or approximately the same diameter as the flexible hose (14) with which it is obliquely connected.

8. Device according to claim 7, characterised in that the first channel (21) is straight and the means of insertion of the graft in the scalp comprise a first gun (30) able to co-operate with the internal bore of the second needle, arranged to push the said graft through the second needle.

9. Device according to any of claims 7 and 8, characterised in that the oblique connection (23) is inclined from the front towards the back of the second needle at an angle of between the order of 10° and the order of 60°.

10. Device according to any of claims 5 to 9, characterised in that the means of insertion are pneumatic.

11. Device according to any of claims 5 to 9, characterised in that the means of insertion are manual.

12. Device according to any of claims 5 to 11, characterised in that the device also has means (31, 34) of thrust against the scalp arranged to cause the withdrawal of the second needle (20) after insertion and deposit of the graft in the scalp.

13. Device according to claim 12 dependent on claim 8, characterised in that the means of thrust comprise a second channel (22) parallel to the first channel (21) and a second piston (31) able to rest on the scalp, the second piston being of one piece with the first piston.

## Patentansprüche

1. Vorrichtung zur Implantation von Haartransplantat kleinen Durchmessers, umfassend ein Handgerät zum Schneiden, bestehend aus einem Werkzeughalterteil (1), einem in Drehung versetzbaren zylindrischen Werkzeug (2) und einer Antriebsanordnung (3), die dazu dient, das genannte Werkzeug gegenüber dem genannten Halter in Drehung zu versetzen, welches Werkzeug mit einem Hohlrand (6) zum Ausschneiden eines Transplantats versehen ist, dadurch gekennzeichnet, daß das zylindrische Werkzeug eine Hohlnadel darstellt, durch die eine Axialbohrung gleichen Durchmessers wie der schneidende Hohlrand, von dem sie ausgeht, hindurchführt, und dadurch, daß die Vorrichtung Mittel (8, 14, 38, 39) zum Entnehmen des Transplantats durch Absaugen des genannten Transplantats durch die Axialbohrung der Nadel aufweist, welche Entnahmemittel mit einer Sammelschlauchleitung (14) versehen sind, die an einen Behälter (21) zur vorübergehenden Aufnahme der entnommenen Transplantate angeschlossen ist.

2. Vorrichtung nach Anspruch 1, dadurch gekennzeichnet, daß die genannte Sammelschlauchleitung (14) den gleichen Innendurchmesser oder im wesentlichen den gleichen Innendurchmesser hat wie die Bohrung der Nadel, und dadurch, daß die Entnahmevorrichtung Mittel (8,9) zum luftdichten Anschließen der genannten Sammelschlauchleitung an das hintere Ende (7) der rotierenden Nadel, sowie eine Einrichtung (38, 39) zum Anlegen von Unterdruck an die Nadelbohrung und die Sammelschlauchleitung aufweist.

3. Vorrichtung nach Anspruch 2, dadurch gekennzeichnet, daß die Verbindungsmittel (8,9) eine in dem Halterteil fest angeordnete Leitung (8) aufweisen, die an einer Seite (13) axial mit der Sammelschlauchleitung (14) verbunden ist, wobei das hintere Ende (7) der Nadel mit dem anderen Ende der genannten Leitung drehbar verbunden und gegen dieses Ende abgedichtet ist, welche genannte Leitung eine Innenbohrung hat, deren Durchmesser mit dem Innendurchmesser der Sammelschlauchleitung übereinstimmt.

4. Vorrichtung nach einem der vorhergehenden Ansprüche, dadurch gekennzeichnet, daß die Nadel (2) abnehmbar an dem Schneidgeräthalter angebracht ist.

5. Vorrichtung nach einem der vorhergehenden Ansprüche, dadurch gekennzeichnet, daß sie außerdem ein Handgerät zum Implantieren des entnommenen Transplantats aufweist, welches Gerät umfaßt:
einen Hauptteil (15),
den Behälter (21) zur vorübergehenden Aufnahme von Transplantat,
eine zweite Hohlnadel (20) zum Reimplantieren, durch welche Nadel eine Bohrung hindurchführt, die den gleichen Durchmesser hat wie die Bohrung der ersten Hohlnadel,
sowie eine Einrichtung (18, 30, 36) zum Einsetzen des Transplantats in die Kopfhaut durch die genannte zweite Hohlnadel hindurch, entnommen aus dem Behälter zur vorübergehenden Aufnahme.

6. Vorrichtung nach Anspruch 5, dadurch gekennzeichnet, daß der Behälter (21) zur vorübergehenden Aufnahme des Transplantats einen ersten Kanal (21) aufweist, der axial an die zweite Hohlnadel angeschlossen ist.

7. Vorrichtung nach Anspruch 6, dadurch gekennzeichnet, daß der erste Kanal (21) zylindrisch ist, von gleichem Durchmesser oder im wesentlichen gleichem Durchmesser wie die Schlauchleitung (14), die schräg zu ihm hin geführt ist.

8. Vorrichtung nach Anspruch 7, dadurch gekennzeichnet, daß der erste Kanal (21) geradlinig ist, und daß die Mittel zum Einsetzen des Transplantats in die Kopfhaut einen mit der Innenbohrung der zweiten Nadel zusammenwirkenden ersten Kolben (30) aufweisen, der dazu dient, das genannte Transplantat durch die zweite Nadel hindurchzuschieben.

9. Vorrichtung nach Anspruch 7 oder 8, dadurch gekennzeichnet, daß die schräg geführte Verbindung (23) eine zwischen etwa 10° und etwa 60° betragende Neigung von vorn in Richtung auf das hintere Ende der zweiten Nadel aufweist.

10. Vorrichtung nach einem der Ansprüche 5 bis 9, dadurch gekennzeichnet, daß zum Einsetzen des Transplantats eine mit Druckluft arbeitende Einrichtung dient.

11. Vorrichtung nach einem der Ansprüch 5 bis 9, dadurch gekennzeichnet, daß zum Einsetzen des Transplantats eine manuell betätigte Einrichtung dient.

12. Vorrichtung nach einem der Ansprüche 5 bis 11, dadurch gekennzeichnet, daß die Vorrichtung außerdem an die Kopfhaut anlegbare Mittel (31,34) aufweist, die dazu dienen, den Rücklauf der zweiten Nadel (20) nach dem Einsetzen und dem Ablegen des Transplantats in die Kopfhaut auszulösen.

13. Vorrichtung nach Anspruch 12, abhängig von Anspruch 8, dadurch gekennzeichnet, daß die anlegbaren Mittel einen parallel zu dem ersten Kanal (21) verlaufenden zweiten Kanal (22) und einen zweiten Kolben (31) aufweisen, der auf die Kopfhaut führbar ist, wobei der zweite Kolben mit dem ersten Kolben baulich verbunden ist.
